Europäisches Patentamt

(19) European Patent Office

Office européen des brevets

(11) Veröffentlichungsnummer: **0 008 092**
**B1**

(12) **EUROPÄISCHE PATENTSCHRIFT**

(45) Veröffentlichungstag der Patentschrift:
28.07.82

(21) Anmeldenummer: 79102797.2

(22) Anmeldetag: 03.08.79

(51) Int. Cl.³: **C 12 P 1/00,** C 12 N 5/00,
C 12 N 15/00 // A61K39/395,
C12R1/91

(54) Verfahren zur Gewinnung von Immunglobulin-E-Antikörpern der Maus, die hierfür verwendbaren Hybridzellinien und deren Herstellung; Verwendung der so erhaltenen Immunglobulin-E-Antikörper.

(30) Priorität: 10.08.78 DE 2835272
21.06.79 DE 2925388

(43) Veröffentlichungstag der Anmeldung:
20.02.80 Patentblatt 80/4

(45) Bekanntmachung des Hinweises auf die Patenterteilung:
28.07.82 Patentblatt 82/30

(84) Benannte Vertragsstaaten:
AT BE CH DE FR GB IT LU NL SE

(56) Entgegenhaltungen:
DE-A-2 755 802
DE-A-2 826 075

CHEMICAL ABSTRACTS, Band 88, Nr. 7, 13. Februar 1978, Seite 390.

CHEMICAL & ENGINEERING NEWS, Band 57 (1), Seiten 15–17 (1979).

J. IMMUNOL., Band 114 (2), Seiten 615–20 (1975).

(73) Patentinhaber: **SCHERING AKTIENGESELLSCHAFT**
**Berlin und Bergkamen,**
**Müllerstrasse 170/178 Postfach 65 03 11,**
**D-1000 Berlin 65 (DE)**

(72) Erfinder: **Böttcher, Irmgard, Dr.,** Rodelbahnpfad 5,
**D-1000 Berlin 28 (DE)**
Erfinder: **Kapp, Joachim-Friedrich, Dr.,**
**Ludolfingerweg 51, D-1000 Berlin 28 (DE)**
Erfinder: **Ovary, Zoltan, Prof. Dr.,** 1st
**Avenue, 343 East 30. Street 10016, New York (US)**

Verfahren zur Gewinnung von Immunglobulin-E-Antikörpern der Maus, die hierfür verwendbaren Hybrid-Zellinien und deren Herstellung; Verwendung der so erhaltenen Immunglobulin-E-Antikörper

Die Erfindung betrifft neue IgE-produzierende Hybrid-Zellinien, Verfahren zu ihrer Herstellung sowie ihre Verwendung. Die Erfindung betrifft ferner ein Verfahren zur Herstellung bzw. Gewinnung von Immunglobulin-E-(IgE)-Antikörpern der Maus mit bekannter Antigen-Spezifität, mit denen sich an Versuchstieren spezifisch und reproduzierbar lokale und systemische allergische Reaktionen erzeugen lassen.

Allergische Reaktionen werden beim Menschen und bei verschiedenen Tierspezies durch Immunglobuline vom IgE-Typ vermittelt (Ishizaka, K. et al. J. Immunol. 97, 75, 1966). Wegen des extrem niedrigen IgE-Gehalts im Serum allergischer Tiere und Patienten (ng/ml) ist die IgE-Isolierung aus diesem Material ausserordentlich schwierig. Hochgereinigtes IgE wurde aus nur äusserst selten spontan auftretenden IgE-produzierenden Myelomen von Mensch und Ratte isoliert (S.G.O. Johansson et al. Immunology 13, 381, 1967; H. Bazin et al. J. Nat. Cancer Inst. 51, 1359, 1973). Jedoch ist die Allergenspezifität dieser IgE-Myelomproteine weiterhin unbekannt. IgE-produzierende Myelome der Maus sind bisher nicht bekannt.

Weitere Angaben zum Stand der Technik sind in DE-A-2 755 802; Eur. J. Immonol. 1977, 7 (10), 684–90; J. Exp. Med. 1977, 146 (6), 1534–48; J. Immunol., Band 114 (2), Seiten 615–20 (1975); DE-A-2 826 075; Chemical & Engineering News, Band 57 (1), Seiten 15–17 (1979) enthalten.

Für Untersuchungen über den Ursprung und Mechanismus allergischer Prozesse wäre ein homogener IgE-Antikörper mit bekannter und reproduzierbarer Antigen-Spezifität ebenso von Bedeutung wie für die Auffindung von antiallergisch wirksamen Substanzen.

Die permanente Produktion monoklonaler IgE-Antikörper mit bekannter Allergen- oder Haptenspezifität ist bisher nicht bekannt.

In der vorliegenden Erfindung wird erstmals die permanente Produktion muriner IgE-Antikörper mit bekannter Allergenspezifität (Ovalbumin, abgekürzt OA) oder bekannter Haptenspezifität (DNP) durch neu hergestellte permanent wachsende Hybrid-Zellinien beschrieben.

Die neue IgE-anti-OA-produzierende Zellinie IgE-14-205 wurde durch Zellfusionierung zwischen murinen Myelomzellen und Milzzellen von OA-hyperimmunisierten Tieren ebenso wie die neuen IgE-anti-DNP-produzierenden Zellinien durch Zellfusionierung zwischen murinen Myelomzellen und Milzzellen von Mäusen mit Antikörperproduktion gegen DNP in an sich bekannter Weise hergestellt.

Durch die neuen Zellinien ist es erstmals möglich, grössere Mengen von Maus-IgE zur Verfügung zu stellen, was bisher stets mit erheblichen Schwierigkeiten verbunden war. Die mit Hilfe der neuen Zellinien hergestellten IgE-Antikörper sind die ersten IgE-Antikörper, die eine homogene Antigenspezifität besitzen und damit sich besonders zur Auslösung standardisierter allergischer Reaktionen eignen. Darüber hinaus eignen sie sich aufgrund ihrer Homogenität hervorragend als Agens für die verschiedensten immunochemischen, pathophysiologischen, immungenetischen und pharmakologischen Studien zur Aufklärung des Mechanismus allergischer Reaktionen. In der tierexperimentellen Forschung versteht man unter Versuchstieren alle zu Testzwecken in Frage kommenden Tierspezies. Besonders geeignet für die Auffindung antiallergisch wirksamer Verbindungen sind Maus und Ratte, an denen die durch das erfindungsgemässe Verfahren hergestellten IgE-Antikörper in Verbindung mit dem spezifischen Antigen Ovalbumin reproduzierbare allergische Reaktionen erzeugen können.

Die in der vorliegenden Erfindung beschriebenen murinen IgE-Antikörper mit bekannter Haptenspezifität bieten darüber hinaus folgende Vorteile:

1. Haptene (z.B. das besonders geeignete DNP, 2,6-Dinitro- und 2,4,6-Trinitrophenol) sind chemisch genau definierte niedermolekulare, selbst nichtimmunogene Verbindungen. Monoklonale Antikörper mit anti-Hapten-Spezifität sind deshalb hervorragend geeignet für molekularbiologische Untersuchungen zum Mechanismus der Antigen-Antikörper-Interaktion (Bindungsaffinität, Spezifität, Kompetition u.a.).

2. Zur Auslösung allergischer IgE-vermittelter Reaktionen sind bivalente Allergene essentiell (bridging-Theorie). Da Allergene im allgemeinen hochmolekulare Proteine sind, die percutan nicht resorbiert werden und bei peroraler Applikation entweder nicht resorbiert oder zu nichtallergenen Spaltprodukten degradiert werden, sind bisher keine standardisierten tierexperimentellen Modelle für Nahrungsmittelallergien und percutan ausgelöste Allergien vorhanden. Durch die Verfügbarkeit monoklonaler IgE-anti-DNP-Antikörper ist erstmals die Möglichkeit zur Etablierung solcher Modelle gegeben. Als percutan und gastrointestinal resorbierbare Allergene können z.B. (DNP)$_2$-Aminosäuren verwendet werden.

Das hochgereinigte murine IgE aus der Hybrid-Zellinie IgE-14-205 und aus den IgE-anti-DNP-Hybrid-Zellinien kann ausserdem zur Herstellung von Anti-MausIgE-Antikörpern in verschiedenen Tierspezies verwendet werden.

I. Herstellung der Hybrid-Zellinien
1. Verwendetes Zell- und Tiermaterial:
    1.1 Permanente Myelomzellinien:
    Zur Zellfusionierung wurden eine permanent in vitro wachsende 8-Azaguanin-resistente Myelomzellinie P3-x-63-Ag8 [G. Köhler and C. Milstein, Nature 256, 495 (1975)] oder eine daraus gewonnene Subzellinie P3-x63-Ag8-6-5-3 verwendet. Diese besitzt im Gegensatz zu der IgG$_1$ (K) syn-

thetisierenden Originalzellinie keine eigene Immunglobulinsynthese mehr.

1.2 Milzzellen aus hyperimmunisierten Balb/c-Mäusen

1.2.1 Zur Produktion der IgE-anti-OA-synthetisierenden Hybrid-Zellinie IgE-14-205 wurden die Mäuse nach dem Verfahren von Kulczycki et al. [J. Exp. Med. 139, 600 (1974)] immunisiert.

1.2.2 Zur Produktion der IgE-anti-DNP-synthetisierenden Hybrid-Zellinien wurden die Mäuse wie folgt immunisiert: 8 Wochen alte weibliche Balb/c-Mäuse erhielten am Tag 0 1 mg DNP-KLH(Keyhole Limpet Hämocyanin) und 1 mg Aluminiumhydroxyd in 0,5 ml physiologischer Lösung intraperitoneal. Am Tag 10 wurden die Mäuse subcutan injiziert mit 750 Nippostrongylus-brasiliensis Larven (3. Stadium). Am Tag 24 wurden die Tiere geboostert durch eine intraperitoneale Injektion von 1 mg DNP-Nippostrongylus-brasiliensis-Extrakt und 1 mg Aluminiumhydroxyd in 0,5 ml physiologischer Lösung.

2. Zellfusionierung

3–7 Tage nach der letzten Immunisierung werden die Milzzellen mit den Myelomzellen in Gegenwart von 42% Polyäthylenglykol für 1–2 Minuten bei 37° fusioniert (Hämmerling, G.J., Europ. J. Immunol. 7, 743, 1977). Durch HAT-Selektion (Littlefield, J.W., Science 145, 709, 1964) wurden Hybrid-Zellinien gewonnen, deren Zellkulturüberstände 3–5 Wochen nach der Zellfusion im passiven cutanen Anaphylaxietest (PCA) der Ratte auf Antikörperproduktion getestet wurden [PCA zur Auffindung von IgE-anti-OA-Antikörpern nach Ovary, Z., Immunological Methods, et. J.F. Ackroyd, Blackwell Scientific Publ., Oxford, 1964, S. 259; PCA zur Auffindung von IgE-anti-DNP-Antikörpern nach Ovary; Caiazza and Kojima, Int. Arch. Allergy Appl. 5 Immunology 48, 16, 1975; Mota and Wong, Life Sci 8, 813, (1969)]. Nach Auffindung von IgE-Antikörper synthetisierenden Hybrid-Zellinien wurden durch mehrfache Subclonierungen monoclonale Hybrid-Zellinien hergestellt.

II. Permanente Produktion von IgE-Antikörpern mit OA-Spezifität

Nach dem dargestellten Zellhybridisierungsverfahren wurde die monoclonale Hybrid-Zellinie IgE-14-205 hergestellt.

1. IgE-Produktion durch die Hybrid-Zellinie IgE-14-205

1.1 Die Hybrid-Zellinie IgE-14-205 synthetisiert permanent in der Zellkultur (z.B. als Suspensionskultur in DMEM- oder RPMI 1640-Medium) Maus IgE-anti-Ovalbumin-Antikörper, die in das Zellkulturmedium sezerniert werden.

1.2 Die Hybrid-Zellinie IgE-14-205 wächst auch als subcutaner oder intraperitonealer Tumor speziesspezifisch in Balb/c-Mäusen und produziert unter diesen in vivo-Bedingungen grosse Mengen des IgE-Antikörpers, die aus dem Serum und Ascites der tumortragenden Tiere gewonnen werden können (Tab. 1).

Tabelle 1
Wachstum der Hybrid-Zellinie IgE-14-205 als Tumor in der Balb/c-Maus
Quantitativer Nachweis der durch den Tumor produzierten IgE-Antikörper
in Serum und Ascites der tumortragenden Mäuse durch den PCA-Test. Der
PCA-Titer ist als reziproker Wert der höchsten Serum- bzw. Ascitesverdünnung angegeben, der eine allergische Hautreaktion erzeugt.

| IgE-14-205-Applikation | Tage nach Applikation | Tumor-rate | PCA-Titer Serum | Ascites |
|---|---|---|---|---|
| subcutan | 8 | 5/5 | 8 | kein Ascites |
| | 18 | 5/5 | 1 024 | kein Ascites |
| intraperitoneal | 8 | 4/5 | 32 | kein Ascites |
| | 18 | 6/6 | 50 000 | 131 000 |

2. Isolierung des Murinen IgE-Anti-Ovalbumin-Antikörpers der Hybrid-Zellinie IgE-14-205

2.1 Isolierung des IgE-Antikörpers aus dem Zellkulturüberstand der in Zellkultur wachsenden Hybrid-Zellinie IgE-14-105 über folgende Reinigungsschritte:

1. Fraktionierte (NH$_4$)$_2$SO$_4$-Fällung

2. DEAE-Chromatographie mit linearem NaCl-Gradient

3. Sephacryl G200-Gelfiltration

Ausbeute: Einige mg hochgereinigtes biologisch aktives IgE aus 1 l Zellkulturüberstand.

2.2 Isolierung des IgE-Antikörpers aus dem Serum und Ascites der IgE-14-205-Tumor-tragenden Maus. Verfahren gemäss 3.1.

Ausbeute: Einige mg hochgereinigtes biologisch aktives IgE aus 10–20 ml Serum bzw. Ascites.

3. Charakterisierung des IgE-Anti-Ovalbumin-Antikörpers aus der Hybrid-Zellinie IgE-14-205

3.1 Das Molekulargewicht des gereinigten IgE-Antikörpers beträgt ≥ 200 000 daltons (Bestimmung durch Sepharose Gel-Elektrophorese in Gegenwart von Molekulargewichtsmarkern).

3.2 Der IgE-14-205-Antikörper zeigt die für IgE-Immunglobuline typische Hitzelabilität: 2 h 56 °C führen zur biologischen Inaktivierung des Antikörpers (Nachweis im PCA-Test s. Tab. 2).

3.3 Der IgE-14-205-Antikörper besitzt Allergen- spezifität. Der IgE-14-205-Antikörper löst eine allergische Reaktion, z.B. die PCA-Reaktion nur in Verbindung mit dem spezifischen Allergen (Ovalbumin) aus, sie unterbleibt, wenn Ovalbumin weggelassen oder duch ein anderes Protein, z.B. Rinderserumalbumin (BSA) ersetzt wird (s. Tab. 2).

Tabelle 2
Charakterisierung des IgE-14-205-Antikörpers

| IgE-Antikörper | Allergen | PCA-Reaktion |
|---|---|---|
| 1. IgE-14-205 | OA | + |
| 2. IgE-14-205, hitzeinaktiviert (2 h, 56°C) | OA | − |
| 3. IgE-14-205 | − | − |
| 4. − | OA | − |
| 5. IgE-14-205 | BSA | − |

3.4 Der IgE-14-205-Antikörper zeigt die für Maus-Immunglobulin E typische heterologe Reaktion mit Rattenmastzellen. Er löst in der Ratte in Verbindung mit dem spezifischen Allergen allergische Reaktionen aus, z.B. erzeugt der murine IgE-14-205-Antikörper in der Ratte eine passive cutane (PCA) und passive peritoneale Anaphylaxie (PPA) (Tab. 3).

Tabelle 3
Passive peritoneale Anaphylaxie der Ratte, ausgelöst durch den Antikörper IgE-14-205

| Versuchsgruppe | Tierzahl | allergischer Histamin-Release | |
|---|---|---|---|
| | | (IU) | % |
| Kontrolle mit IgE-14-205 | 8 | 1,58 ± 0,31 | 100 |
| Kontrolle ohne IgE-14-205 | 9 | 0,57 ± 0,05 | 0 |
| 5 mg/kg DSCG | 5 | 0,74 ± 0,19 | 17 |

Wistar-Ratten erhielten i.p. 2 ml 1:5 verdünnten Ascites aus IgE-14-205-tumortragenden Mäusen (PCA-Titer 3000). Nach 2 Stunden wurde die allergische Reaktion durch i.v.-Injektion von OA ausgelöst und die allergische Histaminfreisetzung aus den peritonealen Mastzellen spektrofluorimetrisch bestimmt (IU = spektrofluorimetrische Intensitätseinheiten). Hemmung des allergischen Histamin-Release durch 5 mg/kg Disodiumcromoglycat (DSCG, Intal®), i.p. verabreicht direkt vor der OA-Applikation.

4. Verwendung des IgE-14-205-Antikörpers zur Testung antiallergisch wirksamer Verbindungen

Gegenüber den für experimentell erzeugte allergische Reaktionen bisher verwendeten durch Hyperimmunisierung hergestellten IgE-haltigen Immunseren bietet der Einsatz des Antikörpers IgE-14-205 folgende Vorteile:

4.1 Der IgE-14-205-Antikörper ist monoclonal und ermöglicht deshalb jederzeit die Induktion qualitativ und quantitativ reproduzierbarer allergischer Reaktionen.

4.2 Der IgE-14-205-anti-OA-Antikörper ist im Gegensatz zu IgE-haltigen Immunseren frei von Antikörpern anderer Immunglobulinklassen mit gleicher Allergenspezifität (z.B. IgG-anti-OA).

4.3 Der IgE-14-205-anti-OA-Antikörper ist im Gegensatz zu IgE-haltigen Immunseren frei von IgE-Antikörpern mit unbekannter Allergenspezifität.

Der IgE-14-205-Antikörper eignet sich deshalb besonders zur Testung antiallergisch wirksamer Verbindungen, deren Wirkstärke quantitativ reproduzierbar ermittelt werden muss. Tab. 3 zeigt als Beispiel die Hemmung der durch den IgE-14-205-Antikörper ausgelösten passiven peritonealen Anaphylaxie der Ratte durch Disodiumcromoglycat (Intal®).

III. Permanente Produktion von IgE-Antikörpern mit DNP-Spezifität

Nach dem dargestellten Zell-Hybrydisierungsverfahren werden monoclonale Hybrid-Zellinien mit IgE-anti-DNP-Antikörperproduktion hergestellt.

Frequenz von IgE-anti-DNP produzierenden Hybridclones

| Fusions-Nr. | Tage nach der letzten Immunis. | Totale Anzahl von Hybriden | IgE-anti-DNP-Antikörper |
|---|---|---|---|
| 49 | 3 | 34 | 1 |
| 52 | 5 | 187 | 7 |
| 53 | 3 | 538 | 59 |
| 55 | 3 | 238 | 13 |
| | | 997 | 80 |

1. IgE-anti-DNP-Produktion durch die Hybrid-Zelllinien

1.1 Die Hybrid-Zelllinien synthetisieren permanent in der Zellkultur (z.B. als Suspensionskultur in DMEM- oder RPMI 1640-Medium) Maus IgE-anti-DNP-Antikörper, die in das Zellkulturmedium sezerniert werden (Tab. 1).

1.2 Die Hybrid-Zelllinien wachsen auch als subcutane oder intraperitoneale Tumoren speziesspezifisch in Balb/c-Mäusen und produzieren unter diesen in vivo-Bedingungen grosse Mengen monoclonaler IgE-anti-DNP-Antikörper, die aus dem Serum und Ascites der Tumor tragenden Tiere gewonnen werden können (Tab. 1).

Tabelle 1
Antikörperproduktion in vitro und in vivo durch IgE-anti-DNP produzierende Hybrid-Zelllinien
Quantitativer Nachweis der IgE-anti-DNP-Antikörper im PCA-Test.
Der PCA-Titer ist als reziproker Wert der höchsten Verdünnung angegeben, die eine allergische Hautreaktion auslöst. ·

| Hybridom | PCA-Titer Zellkultur-überstand | Serum* | Ascites* |
|---|---|---|---|
| IgE à DNP 53-569 | ≧1000–2000 | >1 000 000 | >1 000 000 |
| IgE à DNP 49-15 | >500 | >1 000 000 | >1 000 000 |
| IgE à DNP 55-82 | >500 | >1 000 000 | >1 000 000 |

\* Serum und Ascites von tumortragenden Balb/c-Mäusen bei massivem intraperitonealem Tumorwachstum.

2. Isolierung der murinen IgE-anti-DNP-Antikörper

2.1 Isolierung der IgE-anti-DNP-Antikörper aus dem Zellkulturüberstand der in Zellkultur wachsenden Hybrid-Zelllinien über folgende Reinigungsschritte:

a) Fraktionierte $(NH_4)_2SO_4$-Fällung
b) DEAE-Sepharose-Chromatographie
c) Sephacryl S 200-Gelfiltration
d) Hydroxyapatit-Chromatographie
e) Präparative Isoelektrofocusierung

Ausbeute: 3 mg hochgereinigtes monoclonales biologisch aktives IgE aus 1 l Zellkulturüberstand.

2.2 Isolierung der IgE-anti-DNP-Antikörper aus dem Serum und Ascites der tumortragenden Maus.

Verfahren gemäss III.1.

Ausbeute: 5 mg hochgereinigtes biologisch aktives IgE aus 10–20 ml Serum bzw. Ascites.

3. Charakterisierung der monoclonalen IgE-anti-DNP-Antikörper

3.1 Die IgE-anti-DNP-Antikörper zeigen die für IgE-Immunglobuline typische Hitzelabilität: 2 Std. 56°C führen zur biologischen Inaktivierung der Antikörper (Nachweis im PCA-Test Tab. 2).

Tabelle 2
Spezifität der monoklonalen IgE à DNP Antikörper

| IgE à DNP-Zellkultur-überstand | Allergen | PCA-Titer |
|---|---|---|
| + | DNP-BSA | 500 |
| + | DNP-Edestin | 500 |
| + | $(DNP)_2$-Aminosäuren | 500 |
| + | $(DNP)_1$-Aminosäuren | 0 |
| + | BSA | 0 |
| − | DNP-BSA | 0 |
| hitzeinaktiviert 4 h, 56°C | DNP-BSA | 50 |

3.2 Die IgE-anti-DNP-Antikörper besitzen Haptenspezifität (Tab. 2). DNP, gebunden an verschiedene Carrierproteine, z.B. Rinderserumalbumin (BSA) oder Edestin, löst eine allergische Reaktion (PCA) aus. Aminosäuren mit 2 DNP-Gruppen pro Molekül lösen ebenfalls eine allergische Reaktion aus. Aminosäuren mit einer DNP-Gruppe pro Molekül sind nicht allergen (Bridging Theorie).

3.3 Die IgE-anti-DNP-Antikörper sind präzipitierbar mit anti-Maus-IgE-Antiserum.

3.4 Die IgE-anti-DNP-Antikörper zeigen die für Maus-Immunglobuline typische heterologe Reaktion mit Rattenmastzellen. Sie lösen in der Ratte in Verbindung mit dem spezifischen Hapten allergische Reaktionen aus (PCA-Test). Aus peritonealen Rattenmastzellen, die in vitro mit IgE-anti-DNP sensibilisiert werden, wird durch anti-Maus-IgE-Antiserum Histamin freigesetzt (Abb. 1).

Abb.1. Allergischer Histamin-Release in vitro

Peritoneale Rattenmastzellen wurden in vitro mit verschiedenen Verdünnungen von IgE à DNP 53-569-Ascites sensibilisiert. Der Histaminrelease wurde durch Kaninchen anti-Maus-IgE-Antiserum (anti-IgE) induziert.

●—● IgE + anti-IgE
△—△ IgE-Kontrolle
■ anti-IgE-Kontrolle

4. Verwendung der IgE-anti-DNP-Antikörper zur Testung antiallergisch wirksamer Verbindungen

Gegenüber den für experimentell erzeugte allergische Reaktionen bisher verwendeten durch Hyperimmunisierung hergestellten IgE-haltigen Immunseren, bietet der Einsatz der IgE-anti-DNP-Antikörper folgende Vorteile:

4.1 Die IgE-anti-DNP-Antikörper sind monoclonal und ermöglichen deshalb jederzeit die Induktion qualitativ und quantitativ reproduzierbarer allergischer Reaktionen.

4.2 Die IgE-anti-DNP-Antikörper sind im Gegensatz zu IgE-haltigen Immunseren frei von Antikörpern anderer Immunglobulinklassen mit gleicher Haptenspezifität (z.B. IgG₁-anti-DNP).

4.3 Die IgE-anti-DNP-Antikörper sind im Gegensatz zu IgE-haltigen Immunseren frei von IgE-Antikörpern mit unbekannter Allergenspezifität.

Die IgE-anti-DNP-Antikörper eignen sich deshalb besonders zur Testung antiallergischer Verbindungen, deren Wirkstärke quantitativ reproduzierbar ermittelt werden muss.

Darüber hinaus bieten die IgE-anti-DNP-Antikörper den Vorteil, dass erstmals tierexperimentelle Modelle für Nahrungsmittelallergien und perkutan ausgelöste Allergien entwickelt werden können.

Am 16. Juli 1979 wurden bei der American Type Culture Collection (ATCC) in Rockville, Md., USA folgende Zellinien hinterlegt:
1) murine Myelomzellinie
P3-X63-Ag-6-5-3
als CRL 8008

2) Hybrid-Zellinie IgE-14-205
als CRL 8009
3) (DNP)Hybrid-Zellinie 53-569
als CRL 8010
4) (DNP)Hybrid-Zellinie 49-15
als CRL 8011

**Patentansprüche**

1. Verfahren zur Gewinnung von IgE-Antikörpern der Maus, dadurch gekennzeichnet, dass man in an sich bekannter Weise die IgE-Antikörper-produzierende Hybrid-Zellinie IgE-14-205 mit bekannter Allergen-Spezifität herstellt und nach Wachstum die IgE-Antikörper isoliert.

2. Verfahren zur Gewinnung von IgE-Antikörpern der Maus, dadurch gekennzeichnet, dass man in an sich bekannter Weise IgE-anti-2,4-Dinitrophenol-(DNP)-Hybrid-Zellinien mit bekannter Haptenspezifität herstellt und nach Wachstum die IgE-Antikörper isoliert.

3. Verwendung von aus der Hybrid-Zellinie IgE-14-205 gewonnenen IgE-Antikörpern der Maus zur Auffindung von antiallergisch wirkenden Verbindungen, dadurch gekennzeichnet, dass man an Versuchstieren spezifisch und reproduzierbar Allergien erzeugen kann.

4. Verwendung von aus den IgE-anti-DNP-Hybrid-Zellinien gewonnenen IgE-Antikörpern der Maus zur Auffindung von antiallergisch wirkenden Verbindungen, dadurch gekennzeichnet, dass man an Versuchstieren spezifisch und reproduzierbar Allergien erzeugen kann.

5. Verwendung des aus der Hybrid-Zellinie IgE-14-205 gewonnenen murinen Immunglobulin E zur Herstellung von Anti-Maus-IgE-Antikörpern in verschiedenen Tierspezies.

6. Verwendung des aus IgE-anti-DNP-Hybrid-Zellinien gewonnenen murinen Immunglobulins E zur Herstellung von Anti-Maus-IgE-Antikörpern in verschiedenen Tierspezies.

7. Murine Hybrid-Zellinie IgE-14-205 (CRL 8009).

8. (DNP)Hybrid-Zellinie 53-569 (CRL 8010).

9. (DNP)Hybrid-Zellinie 49-15 (CRL 8011).

**Claims**

1. Process for obtaining IgE antibodies of the mouse, characterised in that, in a manner known per se, the IgE antibody-producing hybrid cell line IgE-14-205 having a known allergen specificity is manufactured and, after growth, the IgE antibodies are isolated.

2. Process for obtaining IgE antibodies of the mouse, characterised in that, in a manner known per se, IgE anti-2,4-dinitrophenol (DNP) hybrid cell lines having a known hapten specificity are manufactured and, after growth, the IgE antibodies are isolated.

3. Use of IgE antibodies of the mouse obtained from the hybrid cell line IgE-14-205 for detecting compounds that have an antiallergic action, characterised in that specific and reproducible allergies can be produced in experimental animals.

4. Use of IgE antibodies of the mouse obtained from the IgE anti-DNP hybrid cell lines for detecting compounds that have an antiallergic action, characterised in that specific and reproducible allergies can be produced in experimental animals.

5. Use of the murine immunoglobulin E obtained from the hybrid cell line IgE-14-205 for the manufacture of anti-mouse-IgE antibodies in various species of animals.

6. Use of the murine immunoglobulin E obtained from IgE-anti-DNP hybrid cell lines for the manufacture of anti-mouse-IgE antibodies in various species of animals.

7. Murine hybrid cell line IgE-14-205 (CRL 8009).

8. (DNP) hybrid cell line 53-569 (CRL 8010).

9. (DNP) hybrid cell line 49-15 (CRL 8011).

**Revendications**

1. Procédé d'obtention d'anticorps de type IgE de souris, caractérisé en ce qu'on produit de façon connue en soi la lignée de cellules hybrides IgE-14-205, produisant les anticorps IgE présentant de la spécificité à l'égard d'allergènes connus et en ce qu'on isole, après la croissance des cellules, les anticorps IgE.

2. Procédé d'obtention d'anticorps IgE de souris, caractérisé en ce qu'on produit de façon connue en soi des lignées de cellules hybrides produisant de l'IgE anti-dinitro-2,4 phénol (DNP) et présentant une spécificité à l'égard d'haptènes connus, et en ce que, après croissance des cellules, on isole les anticorps IgE.

3. Application des IgE, anticorps obtenus à partir de la lignée de cellules hybrides IgE-14-205 de la souris, pour trouver des composés doués d'une action antiallergique, application caractérisée en ce que l'on peut provoquer sur des animaux d'essai des allergies spécifiques et reproductibles.

4. Application des IgE, anticorps de souris obtenus à partir de lignées de cellules hybrides productrices de IgE anti-DNP, pour la découverte de composés doués d'une action antiallergique, application caractérisée en ce qu'on peut provoquer chez les animaux d'essai des allergies spécifiques et reproductibles.

5. Application de l'immunoglobuline E de souris, obtenue à partir de la lignée de cellules hybrides IgE-14-205 à la production de IgE, anticorps antisouris, chez diverses espèces animales.

6. Application de l'immunoglobuline E de souris, obtenue à partir de lignées de cellules hybrides produisant de l'IgE anti-DNP, pour la production de IgE, anticorps anti-souris, dans diverses espèces animales.

7. Lignées de cellules hybrides de souris IgE-14-205 (CRL 8009).

8. Lignées de cellules hybrides (DNP) 53-569 (CRL 8010).

9. Lignées de cellules hybrides (DNP) 49-15 (CRL 8011).